(19) Europäisches Patentamt
European Patent Office
Office européen des brevets

(11) Veröffentlichungsnummer : **0 006 102**
**B1**

(12) # EUROPÄISCHE PATENTSCHRIFT

(45) Veröffentlichungstag der Patentschrift : **11.02.81**

(21) Anmeldenummer : **79100068.0**

(22) Anmeldetag : **11.01.79**

(51) Int. Cl.³ : **C 07 D233/60**, C 07 D233/64// A61K31/415, C08K5/34, C10M1/32

(54) **1-Hydroxymethylimidazole, Verfahren zu ihrer Herstellung und ihre Verwendung als chemische Zwischenprodukte.**

(30) Priorität : **10.06.78 DE 2825547**

(43) Veröffentlichungstag der Anmeldung : **09.01.80 (Patentblatt 80/01)**

(45) Bekanntmachung des Hinweises auf die Patenterteilung : **11.02.81 Patentblatt 81/06**

(84) Benannte Vertragsstaaten : **CH DE FR GB IT**

(56) Entgegenhaltungen : **DE - A - 2 637 670**

(73) Patentinhaber : **BASF Aktiengesellschaft**
**Carl-Bosch-Strasse 38**
**D-6700 Ludwigshafen (DE)**

(72) Erfinder : **Dockner, Toni, Dr.**
**Grossgasse 6**
**D-6701 Meckenheim (DE)**
Erfinder : **Frank, Anton**
**Bannwasserstrasse 72**
**D-6700 Ludwigshafen (DE)**
Erfinder : **Kempe, Uwe, Dr.**
**Moenchhofstrasse 3 b**
**D-6900 Heidelberg (DE)**
Erfinder : **Wetzler, Matthias**
**Liebermannstrasse 7**
**D-6700 Ludwigshafen (DE)**
Erfinder : **Karn, Helmut**
**Sternstrasse 120**
**D-6700 Ludwigshafen (DE)**

**EP 0 006 102 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 17, rue du Louvre, 75001 Paris, France

1-Hydroxymethylimidazole, Verfahren zu ihrer Herstellung und ihre Verwendung als chemische Zwischenprodukte

Die Erfindung betrifft gegebenenfalls in 2-Stellung alkylsubstituierte 1-Hydroxymethylimidazole, ihre Herstellung und ihre Verwendung als chemische Zwischenprodukte.

Es wurde gefunden, daß 1-Hydroxymethylimidazole der Formel 1

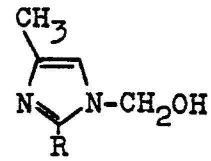

(1)

in der R ein Wasserstoffatom oder einen Alkylrest mit 1 bis 18 C-Atomen bedeutet, chemische Zwischenprodukte mit wertvollen Eigenschaften darstellen.

Von den Verbindungen der Formel 1 sind insbesondere die Verbindungen, in denen R einen Alkylrest mit 1 bis 4 C-Atomen bedeutet, hervorzuheben und als besonders bevorzugt ist die Verbindung, in der R ein Wasserstoffatom bedeutet, zu nennen.

Die Verbindungen der Formel 1 werden hergestellt, indem man ein Imidazol der Formel 2

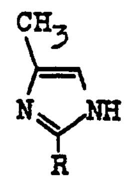

(2)

in der R die oben angegebenen Bedeutungen hat, mit Paraformaldehyd oder Trioxan bei Temperaturen von 40 bis 150 °C direkt oder in Gegenwart eines aromatischen Kohlenwasserstoffs als Lösungsmittel umsetzt.

Die Ausgangsverbindungen werden zweckmäßigerweise in einem Molverhältnis von 0,8 bis 1 : 1 bis 0,8, wobei der Paraformaldehyd oder das Trioxan auf monomeren Formaldehyd bezogen wird und das besonders bevorzugte Molverhältnis bei 1 : 1 liegt, verwendet.

Die bevorzugten Temperaturen liegen bei 50 bis 70 °C. Bei der direkten Umsetzung erfolgt die Reaktion in der Schmelze der Ausgangsstoffe. Wird die Reaktion in Gegenwart eines Lösungsmittels durchgeführt, sind Alkylbenzole, wie Toluol oder Xylol, Chlorbenzol oder Nitrobenzol zweckmäßige Lösungsmittel. In der Regel ist die Reaktion innerhalb von 10 bis 20 Stunden beendet, wenn ein Lösungsmittel verwendet wird, während die direkte Umsetzung in der Schmelze innerhalb von 1 bis 2 Stunden beendet ist.

Das Herstellungsverfahren der direkten Hydroxymethylierung am Stickstoffatom 1 erfolgt in hoher Ausbeute und ohne störende Nebenreaktionen. Dieses Ergebnis ist überraschend und war nicht ohne weiteres voraussehbar. Vielmehr wäre zu erwarten gewesen, daß neben der 5-Hydroxymethylverbindung Dihydrazopyrazin-Derivate nach der in « Chemical and Pharmaceutical Bulletin » 22, 1975, S. 2 359-64 beschriebenen Gleichung

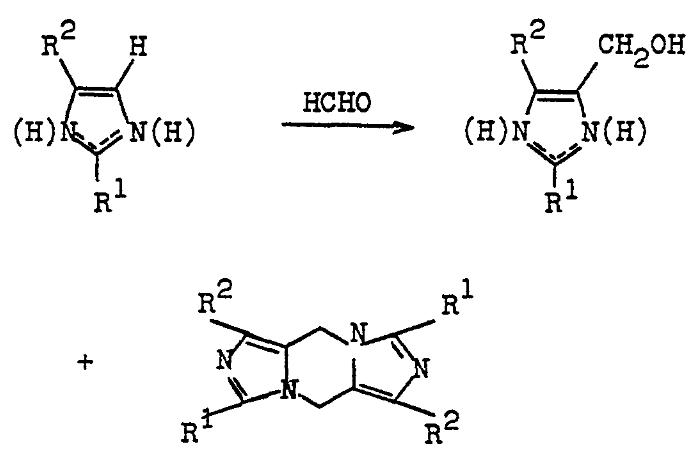


in beträchtlichen Mengen gebildet werden.

Die erfindungsgemäßen Verbindungen sind wertvolle chemische Verbindungen, die beispielsweise für die Härtung von Epoxidharzen, als Emulgatoren für Wasser-Öl-Emulsionen, als Schmiermittelzusätze oder als Zwischenprodukte für Arzneimittel der Human- oder Tiermedizin verwendet werden können.

Das 4-Methyl-1-hydroxymethylimidazol ist eine neue Ausgangsverbindung für die Herstellung von 4-Methyl-5-[(2-aminoäthyl)-thiomethyl]-imidazol, welches ein wichtiges Zwischenprodukt für die Herstellung des Arzneimittels Cimetidin (N-Cyan-N'-methyl-N''-[2-((4-methyl-5-imidazolyl)-methylmercapto)-äthyl]-guanidin) darstellt, wie es beispielsweise in DE-A-23 44 779 und DE-A-26 49 059 beschrieben wird.

Hierzu wird das 4-Methyl-1-hydroxymethylimidazol mit Cysteamin in konzentrierter Salzsäure umgesetzt, indem man 4-Methyl-1-hydroxymethylimidazol und Cysteamin in der 2- bis 5-fach molaren Menge konzentrierter Salzsäure 10 bis 20 Stunden lang zum Sieden erhitzt, die überschüssige Salzsäure unter vermindertem Druck abdestilliert und das so erhaltene rohe 4-Methyl-5-[(2-aminoäthyl)-thiomethyl]-imidazol-dihydrochlorid durch Umkristallisation aus Alkohol reinigt.

Weiterhin lassen sich die 1-Hydroxymethylimidazole der Formel 1 gezielt durch Erhitzen in wäßriger Salzsäure von 10 bis 37, bevorzugt 13 bis 25 Gew. % Chlorwasserstoff in hoher Ausbeute in die entsprechenden 5-Hydroxymethylimidazole umlagern. In wäßriger Salzsäure tritt in dem bevorzugten Bereich praktisch keine Chlormethylierung als Nebenreaktion ein.

Diese Umlagerungsreaktion erfolgt bei Normaldruck durch Erhitzen zweckmäßigerweise auf 80 °C bis zu den Siedetemperaturen der Lösung und ist in der Regel nach 10 bis 120 Stunden beendet. Gegebenenfalls ist es zweckmäßig, in einem geschlossenen Gefäß bei geringen Überdruck bis zu 2 bar und bei Temperaturen bis 125 °C zu arbeiten. Gegebenenfalls kann diese Umlagerungsreaktion in wäßrigalkoholischer Chlorwasserstofflösung erfolgen, wobei bis zu 50 Vol % Äthylalkohol zweckmäßig sind.

Diese überraschend glatt verlaufende Umlagerung schafft ein Verfahren zur Herstellung von 5-Hydroxymethylimidazolen der Formel 3

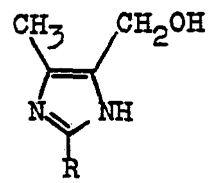

(3)

in der R die oben angegebenen Bedeutungen hat.

Es ist bekannt, daß die 5-Hydroxymethylimidazole unter Umständen nur schwierig hergestellt werden können. Beispielsweise wird das 4-Methyl-5-hydroxymethylimidazol aus 4-Methylimidazol-5-carbonsäureestern durch Reduktion mit Lithiumaluminiumhydrid [J. Med. Chem. *19*, 923 - 928 (1976)] oder mit Alkalimetallen oder Calcium in flüssigem Ammoniak (DE-A-26 37 670) in recht umständlicher Weise hergestellt.

Das durch Umlagerung aus der 1-Hydroxymethylverbindung erhaltene 4-Methyl-5-hydroxymethylimidazol läßt sich in an sich üblicher Weise mit Cysteamin in siedender konzentrierter wäßriger Salzsäure zum 4-Methyl-5-[(2-aminoäthyl)-thiomethyl]-imidazol, dem bekannten Cimetidin-Zwischenprodukt, umsetzen. In einer besonders vorteilhaften und wirtschaftlicheren Ausführungsform erfolgt anstelle des Cysteamins die Umsetzung mit 2,2-Dimethylthiazolidin, das unter den Reaktionsbedingungen in Aceton und Cysteamin, welches dann reagiert, zerfällt.

## Beispiel 1

1-Hydroxymethyl-4-methylimidazol

410 g (= 5 Mol) 4-Methylimidazol werden in
2 l Toluol auf 50 °C erwärmt. In die Lösung werden
150 g Paraformaldehyd eingetragen, der sich unter schwach exothermer Reaktion (Temperaturanstieg von 50 auf 55 °C) löst. Die Mischung wird 17 Studen lang bei 50 bis 55 °C nachgerührt, im Eisbad auf 0 bis 5 °C abgekühlt, die Kristalle abgesaugt und unter Vakuum getrocknet. Die Ausbeute von 532 g entspricht 95,0 % d. Th., Schmelzpunkt : 65 bis 67 °C.

Elementaranalyse :

| | C | H | N | O |
|---|---|---|---|---|
| Ber. : | 53,55 % | 7,18 % | 24,98 % | 14,27 % |
| gef. : | 53,3 % | 7,3 % | 24,8 % | 15,1 % |

## Beispiel 2

82 g 4-Methylimidazol werden bei 60 bis 65 °C mit
30 g Paraformaldehyd portionsweise versetzt und 1 Stunde bei 60 bis 65 °C belassen, wobei der Paraformaldehyd vollständig gelöst wird. Nach dem Erkalten wird das Reaktionsgemisch

pulverisiert. Man erhält 112 g 1-Hydroxymethyl-4-methylimidazol vom Schmelzpunkt 59 bis 60 °C. Das entspricht der theoretischen Ausbeute.

Beispiel 3

110 g 2-Äthyl-4-methylimidazol werden in
400 ml Toluol bei 50 °C gelöst. In diese Lösung werden unter Rühren
30 g Paraformaldehyd eingetragen. Man rührt 16 Stunden bei 50 bis 55 °C nach, kühlt im Eisbad und saugt den Kristallbrei ab. Das erhaltene Rohprodukt wird aus
300 ml Aceton umkristallisiert. Man erhält 130 g 1-Hydroxymethyl-2-äthyl-4-methylimidazol vom Schmelzpunkt 83,1 bis 86,3 °C entsprechend einer Ausbeute von 92,9 % d. Th.

Beispiel 4

192,0 g 2,4-Dimethylimidazol werden in
1,5 g Toluol gelöst und bei 50 °C mit
60,0 g Paraformaldehyd versetzt. Der Paraformaldehyd geht in Lösung, nach ca. 15 Minuten fällt ein dicker weißer Niederschlag aus. Man rührt noch 30 Minuten bei 50 bis 60 °C nach, kühlt im Eisbad und saugt die Kristalle ab.
Nach Trocknung unter vermindertem Druck erhält man 250 g 1-Hydroxymethyl-2,4-dimethylimidazol vom Schmelzpunkt 110,5 bis 111 °C entsprechend einer Ausbeute von 99,2 % d. Th.

Beispiel 5

4-Methyl-5-hydroxymethylimidazol-hydrochlorid

560 g (= 5 Mol) 1-Hydroxymethyl-4-methylimidazol werden unter Kühlung bei 25 °C in
2 250 g HCl conc. eingetragen und 17 Stunden lang am Rückfluß gekocht. Die Salzsäure wird unter vermindertem Druck möglichst restlos abdestilliert und der Rückstand aus 2,5 l Alkohol umkristallisiert. Man erhält 340 g Kristalle vom Schmelzpunkt 207 bis 214 °C. Das Filtrat wird eingeengt und im Eisbad gekühlt und liefert weitere 192 g Kristalle vom Schmelzpunkt 202 bis 211 °C. Aus dem Restfiltrat werden durch Einengen und Kühlen weitere 48 g Kristalle vom Schmelzpunkt 202 bis 209 °C erhalten.
Die Gesamtausbeute von 580 g 4-Methyl-5-hydroxymethylimidazol-hydrochlorid, das 5 bis 10 % Chlormethylverbindung enthält, entspricht in etwa einer Ausbeute von 71 % der Theorie.

Beispiel 6

2-Äthyl-4-methyl-5-hydroxymethylimidazol

45 g 1-Hydroxymethyl-2-äthyl-4-methylimidazol werden in eine Mischung von
100 g 36 % iger Salzsäure und 100 g Wasser eingetragen und 17 Stunden am Rückfluß gekocht. Die Reaktionslösung wird unter vermindertem Druck eingedampft, der Rückstand aus Äthanol umkristallisiert. Man erhält 39 g 2-Äthyl-4-methyl-5-hydroxymethylimidazolhydrochlorid vom Schmelzpunkt 124,3 bis 125,5 °C entsprechend einer Ausbeute von 68,8 % d. Th.

Beispiel 7

126 g 1-Hydroxymethyl-2,4-dimethylimidazol werden in eine Mischung aus
300 g conc. Salzsäure und 300 g Wasser eingetragen und 17 Stunden lang am Rückfluß gekocht. Danach wird die Salzsäure unter vermindertem Druck abdestilliert, der Rückstand aus Äthanol umkristallisiert. Man erhält 130 g 2,4-Dimethyl-5-hydroxymethylimidazolhydrochlorid vom Schmelzpunkt 237 bis 238 °C, entsprechend einer Ausbeute von 80,0 % d. Th.

Beispiel 8

4-Methyl-5-[(2-aminoäthyl)-thiomethyl]-imidazol-dihydrochlorid

30 g 4-Methyl-5-hydroxymethylimidazol-hydrochlorid und
23 g Cysteamin werden in
450 ml conc. Salzsäure 17 Stunden lang am Rückfluß gekocht. Die Lösung wird unter vermindertem Druck zur Trockene eingedampft. Der Rückstand (61 g) wird aus 500 ml Alkohol umkristallisiert. Man erhält 41 g 4-Methyl-5-[(2-aminoäthyl)-thiomethyl]-imidazol-dihydrochlorid vom Schmelzpunkt 184 bis 191 °C entsprechend einer Ausbeute von 84,0 % d. Th.

Beispiel 9

112 Teile 1-Hydroxymethyl-4-methylimidazol werden mit
1 080 Teilen 15 % iger wäßriger Salzsäure bei 2 bar 48 Stunden lang auf 125 °C erhitzt. Das Reaktionsgemisch wird unter vermindertem Druck eingedampft, der Rückstand (157 Teile) in 500 Teilen Wasser gelöst und mit ca. 50 Teilen 50 % iger Natronlauge auf pH 8,6 gestellt. Die erhaltene Lösung wird filtriert und unter vermindertem Druck eingedampft, der Rückstand (173 Teile) wird mit 250 Teilen Methanol aufgekocht, abgekühlt und abgesaugt. Man wäscht mit 50 Teilen Methanol nach und dampft das Filtrat unter Vakuum ein. Man erhält 120 Teile Eindampfrückstand, die in 240 Teilen 2-Propanol heiß gelöst und filtriert werden. Das Filtrat wird über Nacht auf 0 bis 5 °C gekühlt, die Kristalle abgesaugt und getrocknet. Man erhält 68 Teile 4-Methyl-5-hydroxymethylimidazol vom Schmelzpunkt 122 bis 130 °C. Die Mutterlauge wird unter vermindertem Druck zur Hälfte eingeengt und der Rückstand über Nacht auf 0 bis 5 °C gekühlt. Hierbei scheiden sich weitere 18 Teile 4-Methyl-5-hydroxy-methylimidazol ab. Die Gesamtausbeute beträgt 86 Teile, entsprechend einer Ausbeute von 76,8 % d. Th. Das Produkt kann durch weitere Umkristallisation aus 2-Propanol weiter gereinigt werden. Der Schmelzpunkt der reinen Verbindung beträgt 137 bis 138 °C, und im Dünnschichtchromatogramm erscheint ein Fleck bei $R_F = 0{,}54$.

Dünnschichtchromatographie

Platte : Kieselgel 60 F 254 « Merck »
Laufmittel : 70 Teile Chloroform, 50 Teile Methanol
Entwicklung : Jod-Kammer.

Beispiel 10

4-Methyl-5-[(2-aminoäthyl)-thiomethyl]-imidazol-dihydrochlorid

Zu
1 350 ml conc. Salzsäure werden unter Kühlung bei 20 bis 30 °C
108 g 4-Methyl-5-hydroxymethylimidazol-hydrochlorid (= 20 Mol % Überschuß) und 72 g 2,2-Dimethylthiazolidin eingetragen. Die Mischung wird 17 Stunden am Rückfluß gekocht und dann unter vermindertem Druck zur Trockene eingedampft. Der erhaltene Rückstand (= 235 g) wird aus 400 ml Alkohol umkristallisiert.
Man erhält 144 g 4-Methyl-5-[(2-aminoäthyl)-thiomethyl]-imidazol-dihydrochlorid vom Schmelzpunkt 185 bis 192,5 °C entsprechend einer Ausbeute von 98,1 % d. Th.

Beispiel 11 (Vergleichsbeispiel)

41 g 4-Methylimidazol,
38,5 g Cysteamin und
15 g Paraformaldehyd werden in 240 ml Salzsäure conc. 17 Stunden am Rückfluß gekocht. Die Salzsäure wird unter vermindertem Druck abdestilliert und der Rückstand aus Alkohol umkristallisiert. Die Ausbeute beträgt 47 g Thiazolidinhydrochlorid vom Schmelzpunkt 135 bis 155 °C, das nach Elementaranalyse und NMR-Spektroskopie ca. 10 % 4-Methyl-5-[(2-aminoäthyl)-thiomethyl]-imidazol-dihydrochlorid enthält.
Das Vergleichsbeispiel zeigt, daß bei dieser Umsetzung die gewünschte Verbindung nur in geringer Menge entsteht.

Beispiel 12

82 g 4-Methylimidazol werden bei 50 °C unter Rühren mit

30 g Paraformaldehyd portionsweise versetzt. Nach 1 Stunde ist der Paraformaldehyd restlos gelöst. Die Schmelze wird in eine Lösung von
113 g Cysteaminhydrochlorid in
450 g conc. Salzsäure eingetropft und die Mischung 17 Stunden lang am Rückfluß gekocht. Anschliepend wird unter vermindertem Druck eingedampft und der erhaltene Rückstand zweimal aus Äthanol umkristallisiert.
Man erhält 160 g 4-Methyl-5-[(2-aminoäthyl)-thiomethyl]-imidazol-dihydrochlorid vom Schmelzpunkt 185 bis 192 °C entsprechend einer Ausbeute von 65,5 % d. Th.

Beispiel 13

90 g 4-Methyl-5-hydroxymethylimidazol-hydrochlorid (0,6 Mol) und
70 g 2,2-Dimethylthiazolidin (technische Qualität, 78,8 % ig, 0,47 Mol) werden unter Kühlung bei 20 °C in 1 350 ml conc. wäßriger Salzsäure eingetragen. Die Mischung wird 17 Stunden lang unter Rückfluß gekocht, unter vermindertem Druck zur Trockne eingedampft, und der Rückstand wird aus Äthylalkohol umkristallisiert.
Man erhält 106 g 4-Methyl-5-[(2-aminoäthyl)-thiomethyl]-imidazol-dihydrochlorid vom Schmelzpunkt 182,2 bis 191,2 °C, entsprechend einer Ausbeute von 92,4 % d. Th. und bezogen auf 2,2-Dimethyl-thiazolidin. Das IR-Spektrum ist identisch mit der Verbindung des Beispiels 10.

**Ansprüche**

1. 1-Hydroxymethylimidazole der Formel 1

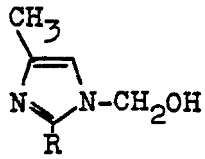

(1)

in der R ein Wasserstoffatom oder einen Alkylrest mit 1 bis 18 C-Atomen bedeutet.

2. 4-Methyl-1-hydroxymethylimidazol.

3. Verfahren zur Herstellung der Verbindungen nach Anspruch 1, dadurch gekennzeichnet, daß man ein Imidazol der Formel 2

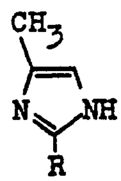

(2)

in der R die in Anspruch 1 angegebenen Bedeutungen hat, mir Paraformaldehyd oder Trioxan bei Temperaturen von 40 bis 150 °C direkt oder in Gegenwart eines aromatischen Kohlenwasserstoffs als Lösungsmittel umsetzt.

4. Verfahren zur Herstellung der Verbindungen der Formel 3,

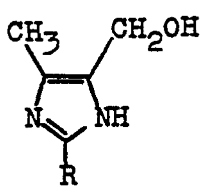

(3)

in der R die in Anspruch 1 angegebenen Bedeutungen hat, dadurch gekennzeichnet, daß man ein Imidazol nach Anspruch 1 durch Erhitzen in wäßriger Salzsäure mit einem Chlorwasserstoff-Gehalt von 10-37 Gew.-% zum 5-Hydroxymethylimidazol umlagert.

5. Verfahren zur Herstellung von 4-Methyl-5-[(2-aminoäthyl)-thiomethyl]-imidazol, dadurch gekennzeichnet, daß man die Verbindung nach Anspruch 2 mit Cysteamin in konzentrierter wäßriger Salzsäure

in der Siedehitze zum 4-Methyl-5[(2-aminoäthyl)-thiomethyl]-imidazol-dihydrochlorid umsetzt.

## Claims

1. A 1-hydroxymethylimidazole of the formula 1

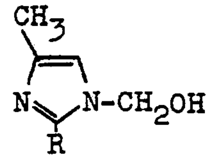

(1)

where R is hydrogen or alkyl of 1 to 18 carbon atoms.

2. 4-Methyl-1-hydroxymethylimidazole.

3. A process for the preparation of a compound as claimed in claim 1, characterized in that an imidazole of the formula 2

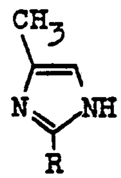

(2)

where R has the meanings given in claim 1, is reacted with paraformaldehyde or trioxane at from 40 to 150 °C, in the absence or presence of an aromatic hydrocarbon as the solvent.

4. A process for the preparation of a compound of the formula 3,

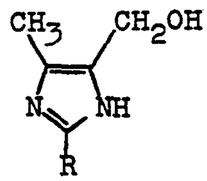

(3)

where R has the meanings given in claim 1, characterized in that an imidazole as claimed in claim 1 is rearranged to 5-hydroxymethylimidazole by heating in aqueous hydrochloric acid having a hydrogen chloride content of 10 to 37 % by weight.

5. A process for the preparation of 4-methyl-5[(2-amino-ethyl)-thiomethyl]-imidazole dihydrochloride, characterized in that the compound of claim 2 is reacted with cysteamine in boiling concentrated aqueous hydrochloric acid to give 4-methyl-5[(2-aminoethyl)-thiomethyl]-imidazole dihydrochloride.

## Revendications

1. 1-Hydroxyméthylimidazoles de formule 1

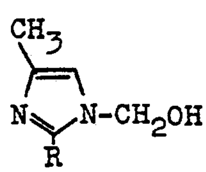

(1)

dans laquelle R désigne un atome d'hydrogène ou un reste alkyle en $C_1$ à $C_{18}$.

2. Le 4-méthyl-1-hydroxyméthylimidazole.

3. Procédé de préparation des composés selon la revendication 1, caractérisé par le fait qu'on fait réagir, à des températures comprises entre 40 et 150 °C, un imidazole de formule 2

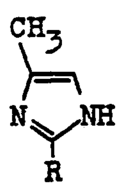

(2)

dans laquelle R a les significations indiquées dans la revendication 1, avec du paraformaldéhyde ou du trioxanne, directement ou en présence d'un hydrocarbure aromatique comme solvant.

4. Procédé de préparation de composés de formule 3

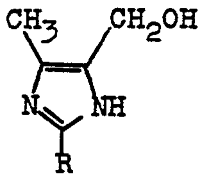

(3)

dans laquelle R a les significations indiquées dans la revendication 1, caractérisé par le fait qu'on transforme un imidazole selon la revendication 1 en 5-hydroxyméthylimidazole par chauffage dans de l'acide chlorhydrique aqueux d'une teneur en acide chlorhydrique comprise entre 10 et 37 % en poids.

5. Procédé de préparation de 4-méthyl-5-[(2-aminoéthyl)-thiométhyl]-imidazole, caractérisé par le fait qu'on fait réagir le composé selon la revendication 2 avec de la cystamine, dans de l'acide chlorhydrique concentré aqueux, à la température d'ébullition, pour obtenir le dichlorhydrate de 4-méthyl-5 [(2-aminoéthyl)-thiométhyl]-imidazole.